(19) Europäisches Patentamt
European Patent Office
Office européen des brevets

(11) EP 1 342 731 A1

(12) **DEMANDE DE BREVET EUROPEEN**

(43) Date de publication:
**10.09.2003 Bulletin 2003/37**

(21) Numéro de dépôt: **03100551.5**

(22) Date de dépôt: **06.03.2003**

(51) Int Cl.[7]: **C08B 5/04**, C08B 13/00, C08B 15/00, C08B 15/06, A61K 7/043, A61K 7/48

(84) Etats contractants désignés:
**AT BE BG CH CY CZ DE DK EE ES FI FR GB GR HU IE IT LI LU MC NL PT RO SE SI SK TR**
Etats d'extension désignés:
**AL LT LV MK**

(30) Priorité: **08.03.2002 FR 0202950**

(71) Demandeur: **L'OREAL**
**75008 Paris (FR)**

(72) Inventeurs:
- **Bernard, Pascale**
**94370 Sucy-en-Brie (FR)**
- **Mondet, Jean**
**93600 Aulnay-sous-Bois (FR)**
- **Ghandchi, Peyman**
**75014 Paris (FR)**
- **Toumi, Béatrice**
**91370 Verrieres-le-Buisson (FR)**

(74) Mandataire: **Poulin, Gérard**
**BREVALEX**
**3, rue du Docteur Lancereaux**
**75008 Paris (FR)**

(54) **Composition cosmétique, notamment de vernis à ongles, comprenant une nitrocellulose modifiée**

(57) La présente invention a pour objet des compositions cosmétiques comprenant au moins un solvant organique et une nitrocellulose modifiée comportant des fonctions hydroxyles libres remplacées, en tout ou partie, par des radicaux -OYR, où R représente une chaîne hydrocarbonée linéaire, ramifiée ou cyclique, de 1 à 500 atomes de carbone, de préférence de 4 à 100 atomes de carbone, saturée ou insaturée, pouvant comporter un ou plusieurs atomes de O, N, S, Si et/ou P, et Y représente une liaison simple ou un groupe de liaison et des procédés de préparation de ladite nitrocellulose.

Ces compositions peuvent être notamment des compositions de vernis à ongles.

EP 1 342 731 A1

## Description

### DOMAINE TECHNIQUE

La présente invention a pour objet une composition cosmétique, notamment de vernis à ongle comprenant au moins une nitrocellulose modifiée, ladite nitrocellulose étant utilisée en tant que filmogène.
La nitrocellulose est un polymère constitué par un assemblage de cycles anhydroglucose nitrées en partie obtenu par estérification d'une partie des fonctions hydroxyles libres d'une cellulose par l'acide nitrique en présence d'acide sulfurique.
Actuellement, la nitrocellulose reste encore le filmogène principal le plus utilisé dans les vernis à ongles à solvants dans des formulations à brillance et tenue optimisées.
Toutefois, les formulations comprenant des nitrocelluloses présentent les inconvénients suivants :

- Elles permettent d'obtenir des films avec un niveau de dureté et de brillance corrects, mais avec une tenue dans le temps non satisfaisante, due notamment à la mauvaise résistance du film à l'écaillage.

- Elles donnent des films durs qui manquent d'adhérence sur l'ongle. On peut remédier à cet inconvénient, en ajoutant des plastifiants mais, dans ce cas, il faut utiliser des quantités très importantes de plastifiants et de co-résines, de l'ordre de celle de la nitrocellulose. De plus, la présence de plastifiants dans ces formulations se traduit, après filmification et séchage, notamment par une évolution des propriétés du film dans le temps, due à la fois à une lente évaporation des solvants résiduels contenus dans le film après séchage et à une perte potentielle d'une partie des plastifiants, notamment par évaporation, d'où un durcissement du film dans le temps et une mauvaise résistance à l'écaillage.

Les recherches effectuées pour remplacer la nitrocellulose par d'autres agents filmogènes tels que des polyacryliques et des polyuréthanes n'ont pas donné de résultats satisfaisants. Aussi, on a envisagé de modifier la nitrocellulose pour améliorer ses propriétés.
A la suite de ces recherches, la demanderesse a découvert de façon surprenante qu'en incorporant une nitrocellulose modifiée de façon adéquate, dans une formulation cosmétique, cela permettait d'obtenir :

- une plastification des films sans recourir à l'adjonction de grandes quantités de plastifiants externes, tout en maintenant un bon niveau de dureté des films ;
- et/ou une augmentation de l'adhérence, par exemple, sur l'ongle, des films de nitrocelluloses modifiées et donc une meilleure résistance à l'écaillage.

Ces résultats sont obtenus par une composition cosmétique comprenant au moins un solvant organique et une nitrocellulose comportant des fonctions hydroxyles libres remplacées, en tout ou partie, par des radicaux -OYR, où R représente une chaîne hydrocarbonée linéaire ou ramifiée comportant de 1 à 500 atomes de carbone, ou cyclique comportant de 3 à 500 atomes de carbone, ladite chaîne étant saturée ou insaturée et pouvant comporter un ou plusieurs atomes de O, N, S, Si et/ou P, et Y représente une liaison simple ou un groupe de liaison.
On précise que, selon l'invention, Y représentant une liaison simple signifie que O et R sont directement liés par une liaison covalente.
Selon l'invention, les chaînes hydrocarbonées R de la nitrocellulose incorporée dans la composition sont liées aux atomes d'oxygène des cycles anhydroglucose de la nitrocellulose, soit par des liaisons simples, soit par l'intermédiaire de groupes de liaison Y pouvant être choisis parmi les groupes -(C=O)-, -(C=O)O-, $-SO_2$, -CO-NH- ou -CO-NR'-, $-Si(R_3)_2-$, les $R_3$ identiques ou différents, étant une chaîne hydrocarbonée linéaire ou ramifiée comportant de 1 à 500 atomes de carbone, ou cyclique comportant de 3 à 500 atomes de carbone, ladite chaîne étant saturée ou insaturée et pouvant comporter un ou plusieurs atomes de O, N, S, Si et/ou P et R' désignant un radical alkyle en C1 à C4. De préférence, $R_3$ comprend de 1 à 10 atomes de carbone.
De préférence dans la nitrocellulose modifiée, selon l'invention, les chaînes hydrocarbonées R comprennent de 4 à 100 atomes de carbone.
Afin de renforcer les capacités d'adhérence de la nitrocellulose modifiée incorporée dans les compositions de l'invention, les chaînes hydrocarbonées R peuvent comporter, en outre, au moins un groupe promoteur d'adhérence.
De préférence, les groupes promoteurs d'adhérence sont situés dans les chaînes hydrocarbonées R latéralement et/ou à leur extrémité.
De préférence, ces groupes sont présents à une teneur inférieure ou égale à 10 % en poids de la nitrocellulose, de préférence inférieure ou égale à 5 %.
A titre d'exemples, les groupes promoteurs d'adhérence peuvent être choisis parmi les groupes :

- hydroxyle -OH ;
- acide carboxylique ou ester $-CO_2R_1$;
- chloro -Cl ;
- amino du type $-NR_1R_2$ avec les $R_1$ et $R_2$, identiques ou différents ;
- pyridino de formule :

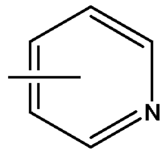

- pyrimidino de formule :

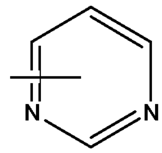

- oxazolino de formules :

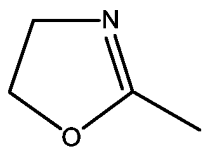
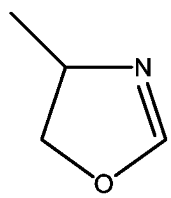
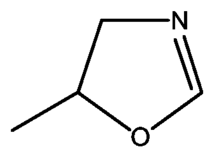

- amido -NH-CO-R' ou $-CO-NH-R_1$;
- pyrrolidono de formules :

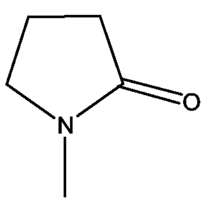
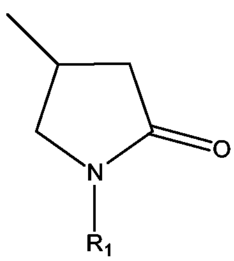
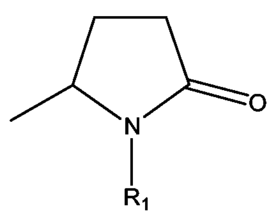
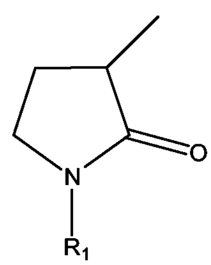

- carbamoyl -O-CO-NH-R' ou -NH-CO-O-R';
- thiocarbamoyl de formule $-O-CS-NHR_1$ ou -NH-CS-O-R' ;
- carbonato -O-CO-O-R';
- uréyl du type $-NR_1-CO-N\ (R_1)\ _2$, les $R_1$ étant identiques ou différents ;
- thiouréyl $-NR_1-CS-N\ (R_1)\ _2$, les $R_1$ étant identiques ou différents ;
- oxamido $-NR_1-CO-CO-N\ (R_1)\ _2$ avec les $R_1$ identiques ou différents ;
- guanidino $-NH-C(=NH)-N(R_1)_2$ avec les $R_1$ identiques ou différents ;
- biguanidino $-NH-C(=NH)-NH-C(=NH)-N(R_1)_2$ avec les $R_1$ identiques ou différents ;
- ester sulfonique $-O-S(=O)_2-R'$;

- sulfonamido $-NR_1-S(=O)_2-R'$ ;
- acétoacétyl $-O-CO-(CH_2)_a-CO-R'$ avec a désignant un nombre entier allant de 1 à 10, de préférence allant de 1 à 5 ;
- siloxane $-Si(R')_{3-b}(OR')_b$ avec b désignant un nombre entier allant de 1 à 3 et R' répondant à la même définition que ci-dessus;
- acétal -OR' ,
  les $R_1$ et $R_2$ désignant H ou un radical alkyle en C1 à C4 et R' désignant un radical alkyle en C1 à C4.

De préférence, selon l'invention, le taux de greffage molaire des radicaux de formule OYR remplaçant, en tout ou partie, les groupes hydroxyles libres par cycle anhydroglucose de la nitrocellulose, selon l'invention, est de 0,1 à 2, préférentiellement de 0,25 à 0,80.
Selon l'invention, les chaînes hydrocarbonées R greffées sur la nitrocellulose, peuvent être de nature polymérique et/ou non polymérique. On précise que, selon l'invention, on entend par chaîne de nature polymérique, une chaîne résultant de la polymérisation d'un ou plusieurs monomères.
En particulier, lorsque les chaînes sont de nature polymérique, elles ne correspondent pas, de préférence, à des chaînes vinyliques et/ou acryliques.
Du point de vue des caractéristiques physico-chimiques, les nitrocelluloses modifiées, selon l'invention, présentent, de préférence, une seule température de transition vitreuse Tg telle que Tg soit inférieure ou égale à 100 °C, et de préférence allant de 30 à 70°C.
La mesure de la température de transition vitreuse (Tg) d'un polymère est effectuée par DMTA (Dynamical and Mechanical Temperature Analysis ou Analyse dynamique et mécanique en température).
Pour mesurer la température de transition vitreuse (Tg) d'un polymère, on effectue des essais de viscoélasticimétrie avec un appareil DMA2980 de T.A. Instruments, sur un échantillon de film de polymère ayant une épaisseur d'environ 200 µm, une largeur de 5 mm et une longueur utile d'environ 10 mm. L'échantillon est préparé par coulage d'une solution du polymère dans une matrice téflonnée puis séchage sur plaque thermostatée à 30 °C pendant 7 jours, sous des conditions d'humidité ambiante (typiquement 50% HR ± 15%). L'échantillon est sollicité en traction, en petites déformations (on lui impose par exemple un déplacement sinusoïdal de ± 8 µm) à la fréquence de 1 Hz lors d'une rampe de température variant par exemple de -50 °C à + 150 °C, avec une variation de température de 3 °C par minute.
On mesure alors le module complexe $E^* = E' + iE''$ du polymère testé en fonction de la température.
De ces mesures, on déduit les modules dynamiques E', E" et le pouvoir amortissant : $tg\delta = E''/E'$.
Puis on trace la courbe des valeurs de tgδ en fonction de la température ; cette courbe peut présenter un pic. La température de transition vitreuse Tg du polymère correspond à la température à laquelle se situe le sommet de ce pic (maximum de la courbe). Cette température n'est pas forcément unique.
De préférence, les nitrocelluloses modifiées, selon l'invention, sont aptes à former un film ayant un module de conservation E' supérieur ou égal à 100 Mpa, notamment allant de 100 Mpa à 5000 Mpa, de préférence supérieur ou égal à 300 Mpa, notamment allant de 300 à 1000 MPa et/ou un pouvoir amortissant tgδ supérieur ou égal à 0,4, notamment allant de 0,4 à 1,5, de préférence supérieur ou égal 0,6, notamment allant de 0,6 à 1, à une température de 30 °C et à une fréquence de 1 Hz.
De préférence, les nitrocelluloses, selon l'invention, sont aptes à former un film ayant une déformation à la rupture $\varepsilon_r$ supérieure ou égale à 5 %, notamment allant de 5 à 500 %, de préférence supérieure ou égale 15%, notamment allant de 15 à 400% et/ou une énergie à rupture par unité de volume $W_r$ supérieure ou égale à 0,2 J/cm$^3$, notamment allant de 0,2 à 100 J/cm$^3$, de préférence supérieure à 1 J/cm$^3$,notamment allant de 1 à 50 J/cm$^3$.
La déformation à la rupture et l'énergie à rupture par unité de volume sont déterminées par des essais de traction effectués sur un film du polymère d'environ 200 µm d'épaisseur. Le film est obtenu par coulage de solution du polymère sur une matrice téflonnée puis séchage sur une plaque thermostatée à 30 °C pendant 7 jours, dans les conditions d'humidité ambiante.
Pour effectuer ces essais, le film est découpé en éprouvettes haltères de longueur utile 33 ± 1 mm et de largeur utile 6 mm. La section (S) de l'éprouvette est alors définie comme : S = largeur x épaisseur (cm$^2$) ; cette section sera utilisée pour le calcul de la contrainte.
Les essais sont réalisés, par exemple, sur un appareil de traction commercialisé sous l'appellation Lloyd® LR5K. Les mesures sont réalisées à température ambiante (20 °C).
Les éprouvettes sont étirées à une vitesse de déplacement de 33 mm/min, correspondant à une vitesse de 100 % d'allongement par minute.
On impose donc une vitesse de déplacement et on mesure simultanément l'allongement ΔL de l'éprouvette et la force F nécessaire pour imposer cet allongement. C'est à partir de ces données ΔL et F que l'on détermine les paramètres contraintes σ et déformation ε.
Il est ainsi obtenu une courbe contrainte $\sigma = (F/S)$ en fonction de la déformation $\varepsilon = (\Delta L/Lo) \times 100$, l'essai étant conduit jusqu'à rupture de l'éprouvette, $L_0$ étant la longueur initiale de l'éprouvette.
La déformation à la rupture $\varepsilon_r$ est la déformation maximale de l'échantillon avant le point de rupture (en %).

L'énergie à rupture par unité de volume Wr en J/cm$^3$ est définie comme la surface sous cette courbe contrainte/déformation telle que :

$$W_r = \int_0^{\varepsilon_r} \sigma.\varepsilon.d\varepsilon$$

Plus particulièrement, les compositions cosmétiques comprenant des nitrocelluloses modifiées telles que définies précédemment peuvent être des compositions de vernis à ongles pour le maquillage des ongles et/ou faux ongles comprenant ladite nitrocellulose modifiée en tant que filmogène. La formulation de ces vernis peut comprendre des pigments, des actifs de soin de l'ongle.

La nitrocellulose modifiée permet de limiter voire de supprimer la charge en plastifiants externes du vernis à ongles, renforçant ainsi l'adhérence et les propriétés mécaniques des revêtements pour donner une meilleure tenue dans le temps.

Dans les compositions cosmétiques suscitées, la teneur en nitrocellulose peut aller de 0,1 % à 60 % en poids, de préférence aller de 1 % à 50 % en poids, et mieux encore de 5 % à 40 % en poids, par rapport au poids total de la composition.

La composition de vernis à ongles peut être employée comme base pour vernis, comme produit de maquillage des ongles, comme composition de finition, encore appelée "top-coat" en terminologie anglosaxonne, à appliquer sur le produit de maquillage des ongles ou bien encore comme produit de soin cosmétique des ongles. Ces compositions peuvent s'appliquer sur les ongles d'êtres humains ou bien encore sur des faux ongles.

Les compositions cosmétiques de l'invention sont des compositions à base de solvant organique.

On peut citer, comme solvant organique, utilisable dans les compositions de l'invention, les solvants suivants :

- les cétones liquides à température ambiante tels que méthyléthylcétone, méthylisobutylcétone, diisobutylcétone, l'isophorone la cyclohexanone, l'acétone
- les alcools liquides à température ambiante tels que l'éthanol, l'isopropanol, le diacétone alcool, le 2-butoxyéthanol, le cyclohexanol ;
- les glycols liquides à température ambiante tels que l'éthylène glycol, le propylène glycol, le pentylène glycol, le glycérol ;
- les éthers de propylène glycol liquides à température ambiante tels que le monométhyléther de propylène glycol, l'acétate de monométhyl éther de propylène glycol, le mono n-butyl éther de dipropylène glycol ;
- les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle ;
- les éthers liquides à température ambiante tels que le diéthyléther, le diméthyléther ou le dichlorodiéthyléther ;
- les alcanes liquides à température ambiante tels que le décane, l'heptane, le dodécane, le cyclohexane ;
- les composés cycliques aromatiques liquides à température ambiante tels que le toluène et le xylène ;
- les aldéhydes liquides à température ambiante tels que le benzaldéhyde, l'acétaldéhyde et
- leurs mélanges.

Le solvant organique peut représenter de 40 à 90 % en poids du poids total de la composition.

La composition à milieu solvant organique peut comprendre en outre de l'eau en très faibles proportions, notamment en une teneur allant de 0,1 à 10 % en poids, par rapport au poids total de la composition, de préférence, moins de 2 % d'eau en poids.

La composition cosmétique, selon l'invention, incorporant la nitrocellulose modifiée ci-dessus, peut comprendre, en outre, un ou plusieurs additifs choisis parmi les polymères filmogènes additionnels, les agents plastifiants, les matières colorantes telles que les pigments, les nacres, les paillettes, les agents épaississants, les agents d'étalement, les agents mouillants, les agents dispersants, les anti-mousses, les conservateurs, les filtres UV, les actifs, les tensioactifs, les cires, les agents hydratants, les parfums, les neutralisants, les stabilisants, les antioxydants.

Ainsi, la composition peut comprendre un polymère filmogène additionnel qui peut être choisi parmi les polymères radicalaires, les polycondensats et les polymères d'origine naturelle.

Le polymère filmogène additionnel peut être choisi notamment dans le groupe formé par les polymères vinyliques, les polyuréthanes, les polyesters, les résines alkydes, les résines époxyesters, les esters de cellulose comme l'acétate de cellulose, l'acétopropionate de cellulose, l'acétobutyrate de cellulose, les résines résultant de la condensation de formaldéhyde avec une arylsulfonamide, et leurs mélanges.

Le polymère filmogène additionnel peut également être une nitrocellulose (non modifiée), ce qui permet l'obtention d'un film de vernis à ongles ayant de bonnes propriétés cosmétiques (notamment de brillance), sans avoir à ajouter de grandes quantités de plastifiants et de co-résines.

Le polymère filmogène additionnel peut être présent en une teneur allant de 0,1 % à 30 % en poids, par rapport au poids total de la composition, et de préférence allant de 1 % à 15 % en poids.

La composition peut comprendre, en outre, au moins un agent plastifiant. En particulier, on peut citer, seuls ou en mélange, les plastifiants usuels, tels que :

- les glycols et leurs dérivés tels que le diéthylène glycol éthyléther, le diéthylène glycol méthyléther, le diéthylène glycol butyléther ou encore le diéthylène glycol hexyléther, l'éthylène glycol éthyléther, l'éthylène glycol butyléther, l'éthylène glycol hexyléther;
- les esters de glycérol,
- les dérivés de propylène glycol et en particulier le propylène glycol phényléther, le propylène glycol diacétate, le dipropylène glycol butyléther, le tripropylène glycol butyléther, le propylène glycol méthyléther, le dipropylène glycol éthyléther, le tripropylène glycol méthyléther et le diéthylène glycol méthyléther, le propylène glycol butyléther,
- des esters d'acides notamment carboxyliques, tels que des citrates, des phtalates, des adipates, des carbonates, des tartrates, des phosphates, des sébaçates,
- des dérivés oxyéthylénés tels que les huiles oxyéthylénées, notamment les huiles végétales telles que l'huile de ricin; et
- leurs mélanges.

La quantité de plastifiant peut être choisie par l'homme du métier sur base de ses connaissances générales, de manière à obtenir une composition ayant des propriétés cosmétiquement acceptables. La teneur en plastifiant peut par exemple aller de 0,1 % à 15 % en poids, par rapport au poids total de la composition, et de préférence de 0,5 % à 10 % en poids.

La composition peut comprendre une matière colorante qui peut être choisie parmi les composés pulvérulents et/ou les colorants solubles dans le milieu de la composition. La matière colorante peut être présente en une teneur allant de 0,001 % à 10 % en poids, par rapport au poids total de la composition. Les composés pulvérulents peuvent être choisis parmi les pigments et/ou les nacres et/ou les paillettes habituellement utilisés dans les vernis à ongles.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique, les pigments métalliques comme l'aluminium ou le bronze. Parmi les pigments organiques, on peut citer le noir de carbone, les pigments de type D & C, et les laques à base de carmin de cochenille, de baryum, strontium, calcium, aluminium, la guanine.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les paillettes peuvent être choisies parmi celles en résine acrylique, polyester, polyéthylène téréphtalate, en aluminium.

Les colorants sont par exemple le rouge Soudan, le DC Red 17, le DC Green 6, le β-carotène, l'huile de soja, le brun Soudan, le DC Yellow, 11, le DC Violet 2, le DC orange 5, le jaune quinoléine. La composition selon l'invention peut en outre comprendre tout additif connu de l'homme du métier comme étant susceptible d'être incorporé dans une telle composition, tels que les agents épaississants, les agents d'étalement, les agents mouillants, les agents dispersants, les anti-mousses, les conservateurs, les filtres UV, les actifs, les tensioactifs, les cires, les agents hydratants, les parfums, les neutralisants, les stabilisants, les antioxydants. Bien entendu, l'homme du métier veillera à choisir ce ou ces éventuels composés complémentaires, et/ou leur quantité, de manière telle que les propriétés avantageuses de la composition pour l'utilisation selon l'invention ne soient pas, ou substantiellement pas, altérées par l'adjonction envisagée.

Parmi les nitrocelluloses modifiées pouvant être incorporées dans les compositions cosmétiques de l'invention, un certain nombre d'entre elles constituent de nouveaux composés particulièrement intéressants pour leurs propriétés filmogènes, leurs résistances à l'écaillage, leurs capacités d'adhérence et leurs qualités de brillance.

De telles nitrocelluloses sont des nitrocelluloses comportant des fonctions hydroxyles libres remplacées, en tout ou partie, par des radicaux -OYR, où R représente une chaîne hydrocarbonée linéaire, ramifiée comportant de 1 à 500 atomes de carbone, ou cyclique comportant de 3 à 500 atomes de carbone, ladite chaîne étant saturée ou insaturée et pouvant comporter un ou plusieurs atomes de O, N, S, Si et/ou P, et Y représente une liaison simple ou un groupe de liaison et ladite chaîne comprenant au moins un groupe promoteur d'adhérence choisi parmi les groupes

suivants :

- chloro -Cl ;
- amino $-NR_1R_2$ avec $R_1$ et $R_2$, identiques ou différents;
- pyridino de formule :

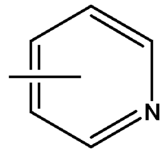

- pyrimidino de formule :

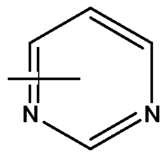

- oxazolino de formules :

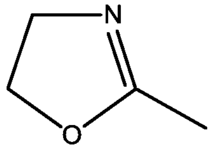
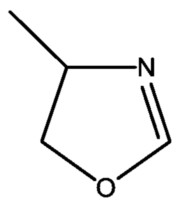
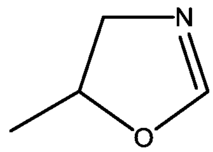

- amido -NH-CO-R' ou $-CO-NH-R_1$ ;
- pyrrolidono de formules :

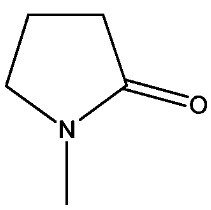
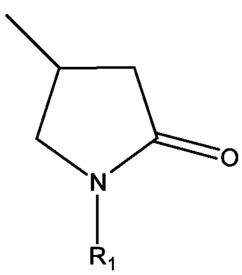
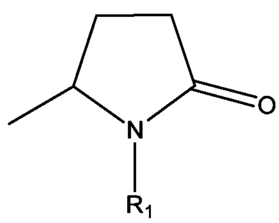
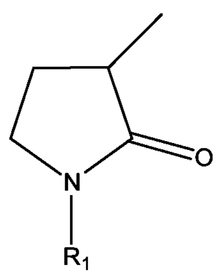

- carbamoyl -O-CO-NH-R' ou -NH-CO-O-R';
- thiocarbamoyl de formule $-O-CS-NHR_1$ ou -NH-CS-O-R'
- carbonato -O-CO-O-R';
- uréyl $-NR_1-CO-N\ (R_1)\ _2$, les $R_1$ étant identiques ou différents ;
- thiouréyl $-NR_1-CS-N\ (R_1)\ _2$, les $R_1$ étant identiques ou différents ;
- oxamido $-NR_1-CO-CO-N\ (R_1)\ _2$ avec les $R_1$ identiques ou différents ;
- guanidino $-NH-C(=NH)-N(R_1)_2$ avec les $R_1$ identiques ou différents ;
- biguanidino $-NH-C(=NH)-NH-C(=NH)-N(R_1)_2$ avec les $R_1$ identiques ou différents ;
- ester sulfonique $-O-S(=O)_2-R'$;
- sulfonamido $-NR_1-S(=O)_2-R'$;
- acétoacétyl $-O-CO-(CH_2)\ _a-CO-R'$ avec a désignant un nombre entier allant de 1 à 10, de préférence allant de 1 à 5 ;

- siloxane -Si-$(R')_{3-b}$ $(OR')_b$ avec b désignant un nombre entier allant de 1 à 3 ;
- acétal -OR',

les $R_1$ et $R_2$ désignant H ou un radical alkyle en C1 à C4 et R' désignant un radical alkyle en C1 à C4.

Selon l'invention, les chaînes hydrocarbonées R de la nitrocellulose selon l'invention sont liées aux atomes d'oxygène des cycles anhydroglucose de la nitrocellulose, soit par des liaisons simples, soit par l'intermédiaire de groupes de liaison Y pouvant être choisis parmi les groupes-(C=O)-, -(C=O)O-, $-SO_2$, -CO-NH- ou -CO-NR' -, -Si $(R_3)_2$-, les $R_3$ identiques ou différents, étant une chaîne hydrocarbonée linéaire, ramifiée comportant de 1 à 500 atomes de carbone, ou cyclique comportant de 3 à 500 atomes de carbone, ladite chaîne étant saturée ou insaturée et pouvant comporter un ou plusieurs atomes de O, N, S, Si et/ou P et R' ayant la même signification que celle donnée précédemment. De préférence, $R_3$ comporte de 1 à 10 atomes de carbone.

De préférence dans la nitrocellulose modifiée, selon l'invention, les chaînes hydrocarbonées R comprennent de 4 à 100 atomes de carbone.

De préférence, les groupes promoteurs d'adhérence sont situés dans les chaînes hydrocarbonées R latéralement et/ou à leur extrémité.

De préférence, ces groupes sont présents à une teneur inférieure ou égale à 10 % en poids de la nitrocellulose, de préférence inférieure ou égale à 5 %.

De préférence, selon l'invention, le taux de greffage molaire des radicaux de formule OYR remplaçant, en tout ou partie, les groupes hydroxyles libres par cycle anhydroglucose de la nitrocellulose, selon l'invention, est de 0,1 à 2, préférentiellement de 0,25 à 0,80.

Selon l'invention, les chaînes hydrocarbonées R greffées sur la nitrocellulose, peuvent être de nature polymérique et/ou non polymérique.

Du point de vue des caractéristiques physico-chimiques, les nitrocelluloses modifiées, selon l'invention, présentent, de préférence, une seule température de transition vitreuse Tg telle que Tg soit inférieure ou égale à 100 °C, et de préférence allant de 30 à 70°C.

De préférence, les nitrocelluloses modifiées, selon l'invention, sont aptes à former un film ayant un module de conservation E' supérieur ou égal à 100 Mpa, notamment allant de 100 Mpa à 5000 Mpa, de préférence supérieur ou égal à 300 Mpa, notamment allant de 300 à 1000 MPa et/ou un pouvoir amortissant tg$\delta$ supérieur ou égal à 0,4, notamment allant de 0,4 à 1,5, de préférence supérieur ou égal 0,6, notamment allant de 0,6 à 1, à une température de 30 °C et à une fréquence de 1 Hz.

De préférence, les nitrocelluloses, selon l'invention, sont aptes à former un film ayant une déformation à la rupture $\varepsilon_r$ supérieure ou égale à 5 %, notamment allant de 5 à 500 %, de préférence supérieure ou égale 15%, notamment allant de 15 à 400% et/ou une énergie à rupture par unité de volume $W_r$ supérieure ou égale à 0,2 J/cm$^3$, notamment allant de 0,2 à 100 J/cm$^3$, de préférence supérieure à 1 J/cm$^3$,notamment allant de 1 à 50 J/cm$^3$.

Les nitrocelluloses de l'invention peuvent être préparées par différents procédés à la portée de l'homme du métier.

En particulier, les nitrocelluloses de l'invention peuvent être préparées par greffage dans un solvant inerte des chaînes hydrocarbonées R tels que définies ci-dessus sur tout ou partie des fonctions hydroxyle libre d'une nitrocellulose de départ.

Les nitrocelluloses de départ utilisées pour introduire les chaînes hydrocarbonées peuvent être des nitrocelluloses filmogènes utilisées habituellement dans les encres, peintures et formulations de vernis à ongles. Ces nitrocelluloses sont préparées industriellement par estérification de la cellulose par un mélange d'acide nitrique et d'acide sulfurique, ce dernier jouant le rôle de déshydratant et déplaçant l'équilibre d'estérification. Elles constituent ainsi des nitrates de cellulose, désignés communément sous l'appellation de nitrocellulose.

De préférence, les nitrocelluloses de départ présentent un pourcentage d'azote de 10 à 13,5% en poids (une cellulose intégralement nitrée ayant un pourcentage d'azote de 14,14 %), ce qui correspond sensiblement à un taux de nitration de 1,7 à 2,5 groupes hydroxyles estérifiés par cycle anhydroglucose sur les 3 groupes -OH du cycle initialement disponibles pour la nitration. En d'autres termes, il reste entre 1 et 0,75 groupe -OH libre, pour le greffage de chaînes hydrocarbonées R, par cycle anhydroglucose de la nitrocellulose.

En ce qui concerne le poids moléculaire des nitrocelluloses de départ, celui-ci est exprimé généralement par la mesure de la viscosité (par chute de bille) d'une solution du polymère (ou collodion) dans un mélange de solvants à un % de polymère donné, ledit pourcentage de polymère étant généralement de 12 à 25 %. En général, le mélange de solvants utilisé pour la caractérisation est constitué de 25 % d'éthanol à 95 % dénaturé, 20 % d'acétate d'éthyle et 55 % de toluène, les pourcentages étant exprimés en poids par rapport au mélange de solvants.

De préférence, les nitrocelluloses de départ, utilisées pour préparer les nitrocelluloses modifiées selon l'invention, ont des viscosités de chute de bille allant de (1/16) à 1000 secondes, de préférence de (1/4) à 100 secondes, pour une concentration en polymère de 12,2 % dans le mélange de solvants cité ci-dessus.

Du point de vue du conditionnement, les nitrocelluloses de départ, sont généralement, du fait de leur caractère

inflammable et de leur pouvoir explosif, présentées :

- soit sous forme mouillée avec, par exemple, 35 % d'alcool (éthanol, isopropanol) ;
- soit en solution, dans un solvant dépourvu d'hydrogène labile;
- soit sous forme de 'chips', c'est-à-dire de mélange comprenant, par exemple, 80 % du polymère et 20 % d'un plastifiant, tel que le dibutylphtalate.

Compte tenu des dangers de manipulation de la nitrocellulose de départ, les procédés de préparation des nitrocelluloses modifiées selon l'invention s'effectueront, dans des conditions douces, à savoir :

- en opérant à une température de réaction de 0 à 80 °C, de préférence, de 20 à 60°C ;
- en opérant, de préférence, en l'absence d'oxygène, pour éviter tout risque d'explosion par contact de la nitrocellulose avec un oxydant, par exemple, en opérant sous un gaz inerte tel que l'argon.

La préparation des nitrocelluloses modifiées selon l'invention peut revêtir différents modes de réalisation, selon la nature de la chaîne hydrocarbonée à greffer.

Ainsi, selon un premier mode de réalisation du procédé selon l'invention, correspondant au cas où les chaînes hydrocarbonées R sont d'origine non polymérique, la réaction de greffage peut s'effectuer dans un solvant inerte par une réaction choisie parmi les réactions d'éthérification, estérification avec un acide carboxylique ou ses dérivés, transestérification avec un ester ou un carbonate, estérification avec un acide sulfonique ou ses dérivés, réaction avec un isocyanate, réaction avec un alcoxysilane.

Pour les parties qui suivent, dans l'exposé des méthodes de préparation de la nitrocellulose modifiée selon l'invention, on utilisera les abréviations suivantes :

- Nitrocell-OH pour la nitrocellulose de départ non modifiée, un seul OH, étant pris en compte, pour la clarté de l'exposé ;
- R pour la chaîne hydrocarbonée à greffer sur la nitrocellulose, répondant à la formule donnée précédemment, c'est-à-dire une chaîne hydrocarbonée linéaire ou ramifiée de 1 à 500 atomes de carbone, ou cyclique de 3 à 500 atomes de carbone, ladite chaîne étant saturée ou insaturée et pouvant comporter un ou plusieurs atomes de O, N, S, Si et/ou P et Y. De préférence, R comporte de 4 à 100 atomes de carbone.

A titre d'exemple, la nitrocellulose de départ peut être représentée par la formule suivante :

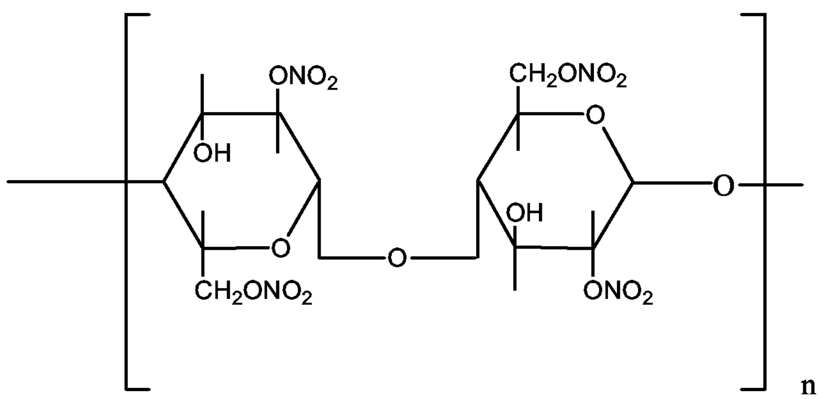

n représentant le nombre d'unités diméres d'anhydroglucose partiellement nitré répétées dans la nitrocellulose. La substitution des OH par des groupes - $ONO_2$ peut être faite aléatoirement sur les -OH primaires et/ou secondaires du cycle anhydroglucose.

Dans la partie ci-après, on va décrire différentes réactions utilisables pour le greffage de chaînes R de nature non polymérique.

1) Ethérification.

Pour l'étherification, les réactions suivantes peuvent être envisagées, réactions pour lesquelles le radical Y faisant la jonction entre les chaînes R et la nitrocellulose est une simple liaison :

- réaction avec un halogénure d'alkyle R-X (X représentant un halogène) :

$$\text{Nitrocell-OH} + \text{R-X} \rightarrow \text{Nitrocell-OR} + \text{HX}$$

Avec X étant un halogène choisi parmi le chlore, le brome ou l'iode.

- réaction avec un époxyde :

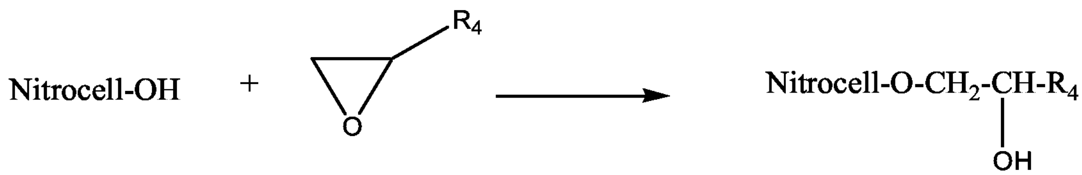

$R_4$ représentant une chaîne rentrant dans la constitution de la chaîne R définie précédemment, ladite chaîne R étant représentée ici par le groupe-$CH_2$-(CHOH) -$R_4$ ;

- réaction avec un aldéhyde en milieu réducteur (tel que le triéthylsilane en présence de platine) :

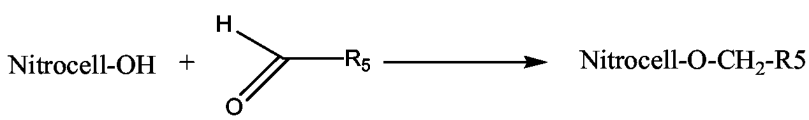

$R_5$ représentant une chaîne rentrant dans la constitution de la chaîne R, ladite chaîne R étant représentée ici par le groupe -$CH_2$-$R_5$.

Selon une variante, cette réaction peut se dérouler en deux étapes, avec pour première étape une réaction préliminaire de l'aldéhyde avec un diol, tel que le glycol, pour former un acétal cyclique :

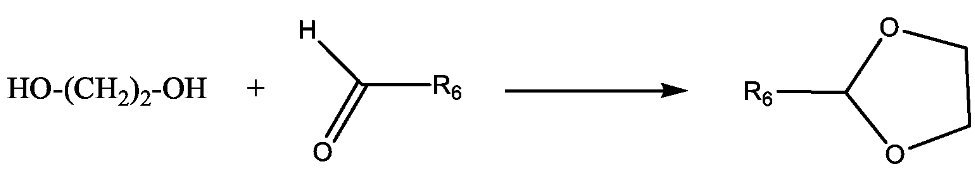

suivi d'une réaction de l'acétal cyclique avec la nitrocellulose :

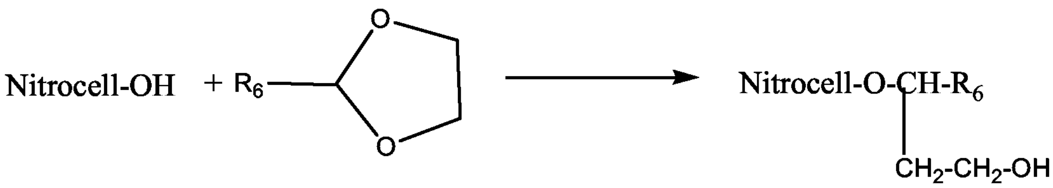

$R_6$ représentant une chaîne entrant dans la constitution de la chaîne R, ladite R chaîne étant représentée ici par le groupe -CH- ($CH_2$-$CH_2$-OH) -$R_6$.

- réaction avec un éther mixte R-O-R' avec R' désignant un radical alkyle en $C_1$-$C_4$, en milieu acide :

$$\text{Nitrocell-OH} + \text{R'-O-R} \rightarrow \text{Nitrocell-O-R} + \text{R'OH}$$

- réaction d'addition des -OH libres de la nitrocellulose sur une double liaison, par exemple terminale, portée par le radical à greffer, en présence de $PdCl_2$ et $HgCl_2$ :

$$\text{Nitrocell-OH} + CH_2{=}CH\text{-}R_7 \rightarrow \text{Nitrocell-O-}CH_2\text{-}CH_2\text{-}R_7$$

ou

$$\text{Nitrocell-OH} + R_aCH{=}CH\text{-}R_b \rightarrow \text{Nitrocell-O-}CHR_a\text{-}CH_2\text{-}R_b$$

$R_7$ représentant une chaîne entrant dans la constitution de la chaîne R, représentée ici par le groupe -$CH_2$-$CH_2$-$R_7$ et $R_a$ et $R_b$ représentant une chaîne entrant dans la constitution de la chaîne R, représentée ici par le groupe -$CHR_a$-$CH_2$-$R_b$.

D'autres réactions d'éthérification peuvent être envisagées, notamment celles mentionnées dans l'ouvrage « Advanced in Organic Chemistry », J.March, John Wiley & Son, Edition 1992.

2) Estérification.

A titre d'exemples pour les réactions d'estérification, on peut citer les réactions suivantes, réactions pour lesquelles Y représente un groupe de liaison CO :

- réaction avec un acide carboxylique R-$CO_2$H :

$$\text{Nitrocell-OH} + \text{R-}CO_2H \rightarrow \text{Nitrocell-O-CO-R} + H_2O$$

- réaction avec un chlorure d'acide R-COCl ou transestérification par un ester R-COOR', telle que :

$$\text{Nitrocell-OH} + \text{R-COCl} \rightarrow \text{Nitrocell-O-CO-R} + \text{HCl}$$

$$\text{Nitrocell-OH} + \text{R-COO-R'} \rightarrow \text{Nitrocell-O-CO-R} + \text{R'-OH}$$

- réaction avec un anhydride d'acide, par exemple :

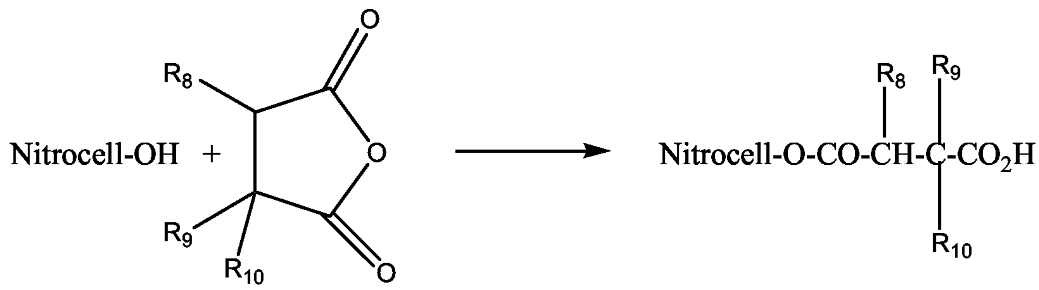

avec $R_8$, $R_9$ et $R_{10}$ étant tels que - $(CHR_8)$ $-CR_9R_{10}-CO_2H$ représente R.

3) Transestérification avec un carbonate.

La réaction suivante avec un carbonate R'-O-CO-O-R peut être envisagée, réaction pour laquelle Y représente un groupe de liaison -CO-O-:

$$\text{Nitrocell-OH} + \text{R'-O-CO-O-R} \rightarrow \text{Nitrocell-O-CO-O-R} + \text{R'-OH}$$

4) Estérification avec un chlorure de sulfonyle.

A titre d'exemples pour les réactions d'estérification avec un acide sulfonique ou un chlorure de sulfonyle, réactions pour lesquelles Y représente un groupe de liaison $-SO_2-$, la réaction suivante peut être envisagée :

$$\text{Nitrocell-OH} + \text{Cl-SO}_2\text{-R} \rightarrow \text{Nitrocell-O-SO}_2\text{-R} + \text{HCl}$$

5) Réaction avec un isocyanate.

A titre d'exemples pour les réactions de formation de liaisons carbamates, réactions pour lesquelles Y représente un groupe de liaison -CO-NH-, la réaction suivante avec un isocyanate OCN-R peut être envisagée :

$$\text{Nitrocell-OH} + \text{OCN-R} \rightarrow \text{Nitrocell-O-CO-NHR}$$

6) Réaction avec un alcoxysilane.

A titre d'exemples pour les réactions de la nitrocellulose avec un alcoxysilane, réactions pour lesquelles Y représente un groupe de liaison $-Si(R_{12})_2-$, la réaction suivante peut être envisagée :

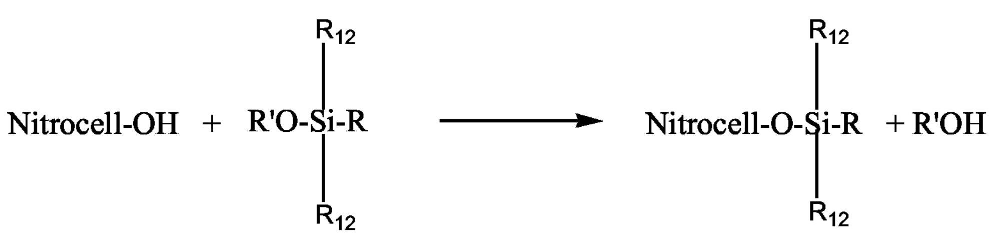

avec les $R_{12}$ identiques ou différents, pouvant être une chaîne hydrocarbonée linéaire ou ramifiée de 1 à 500 atomes de carbone, ou cyclique de 3 à 500 atomes de carbone, ladite chaîne étant saturée ou insaturée et pouvant comporter un ou plusieurs atomes de O, N, S, Si et/ou P, de préférence de 1 à 10 atomes de carbone, les $R_{12}$ ayant donc la même définition que les $R_3$ définis précédemment.

Compte tenu des propriétés générales de la nitrocellulose, les conditions nécessaires aux réactions de greffage d'une chaîne hydrocarbonée de nature non polymère, doivent répondre, de préférence, à un certain nombre de critères.

En effet, la nitrocellulose de départ est un réactif dangereux, du fait de sa très forte inflammabilité et de son pouvoir explosif, ce qui impose une nécessité d'adapter les réactions citées ci-dessus, de faire un choix préférentiel de réactifs et d'appliquer des conditions expérimentales strictes.

Ainsi, pour obtenir un taux de greffage convenable, il est préférable de travailler en milieu solvant homogène, le solvant ou mélange de solvants devant être choisis de manière à pouvoir solubiliser, à la fois, la nitrocellulose de départ, les réactifs de greffage, et de préférence, la nitrocellulose modifiée après greffage des groupements -R.

De plus, le solvant ou mélange de solvants doit être inerte vis-à-vis de la réaction de greffage. En effet, la réaction de greffage se faisant sur les -OH libres de la nitrocellulose, le ou les mélanges de solvants doivent, de préférence, eux-mêmes être exempts de groupes -OH, qui pourraient co-réagir avec les réactifs de greffage, et exempts de groupes différents de -OH mais qui pourraient également réagir avec les réactifs de greffage (tels que les solvants à hydrogène labile, par exemple).

Compte tenu de ce qui a été dit sur les caractéristiques des solvants, les solvants ou mélanges de solvants utilisables pour ces réactions présentent, de façon générale, une polarité, définie par la grandeur $E_T(30)$ définie à partir de l'énergie de transition Z relative au pic de transfert de charge dans le spectre UV du complexe entre l'ion iodure et l'ion 1-méthyl ou 1-éthyl-4-carbométhoxypyridinium avec un $E_T(30)$ de 51,9 et 33,9, tel que cité dans le livre « Advanced in Organic Chemistry » pages 357-361.

Parmi les solvants envisageables, on peut citer l'acétone, la méthyléthylcétone, l'acétate d'éthyle, l'acétate de butyle, le diméthylformamide, le diméthylsulfoxyde, le diméthylacétamide, la pyridine, le tétrahydrofurane, le dioxane, le toluène et leurs mélanges.

De ce fait, vu que une des formes industrielles de livraison de la nitrocellulose est un collodion mouillé avec environ 35 % d'alcool, il sera préférable :

- soit de travailler avec une autre présentation industrielle de la nitrocellulose, tels que les « chips », comprenant 80 % du polymère et 20 % d'un plastifiant tel que le dibutylphtalate ;
- soit de travailler avec une nitrocellulose directement en solution dans un solvant, par exemple, du type acétate, tel que l'acétate d'éthyle ou de butyle;
- soit, enfin, de travailler avec le collodion, ledit collodion étant soumis avant utilisation à une étape d'échange de solvant, par une technique de laboratoire ou industrielle appropriée, telle que la distillation sélective, la distillation azéotropique, l'extraction liquide/liquide, la dialyse.

De préférence, le ou les réactifs de greffage doivent être inertes vis-à-vis de toute réaction sur les groupes esters nitriques de la nitrocellulose, ladite nitrocellulose devant être maintenue en nature et en quantité.

Enfin, compte tenu des dangers de manipulation de la nitrocellulose, le choix préférentiel du type de réactions de greffage et de réactifs se fera, selon l'invention, sur des réactions et réactifs doux, soit :

- en opérant à une température de réaction de 0 à 80 °C, de préférence, de 20 à 60°C ;
- en opérant, de préférence, en l'absence d'oxygène, pour éviter tout risque d'explosion par contact de la nitrocellulose avec un oxydant ;
- le cas échéant, en présence d'un ou plusieurs catalyseurs, adaptés à la réaction pour favoriser les conditions douces.

De préférence, la réaction sera faite sous gaz inerte, tel que l'argon, avec au départ de la réaction, moins de 1 ppm d'oxygène dissous dans le solvant ou mélange de solvants utilisé pour la réaction.

Au regard de toutes ces remarques préliminaires, les réactions préférentielles ainsi que les réactifs associés, remplissant les conditions préférentielles citées ci-dessus, sont choisis, selon l'invention, parmi les réactions suivantes :

- est érification des fonctions hydroxyles par le biais d'un acide carboxylique en présence d'un réactif de couplage tel que le dicyclohexylcarbodiimide ou ses dérivés ;

  A titre d'exemples et de façon non limitative, on peut citer en tant qu'acides carboxyliques l'acide butanoïque, pentanoïque, hexanoïque (ou caproïque), heptanoïque, octanoïque (ou caprylique), éthyl-2-hexanoïque, nonanoïque, décanoïque (ou caprique), néodécanoïque (ou versatique), undécanoïque, dodécanoïque, isononanoïque, et également les acides gras ramifiés en $C_{16}$-$C_{40}$ ou linéaires insaturés en $C_{16}$-$C_{40}$.

- estérification des fonctions hydroxyles par le biais d'un chlorure d'acide carboxylique.

  Par exemple, on peut citer comme chlorures d'acide carboxylique les chlorures des acides mentionnés ci-dessus.

- estérification des fonctions hydroxyles par le biais d'un anhydride d'acide carboxylique ;
  Par exemple, on peut citer comme anhydrides d'acide carboxylique les anhydrides des acides mentionnés ci-dessus.

- réaction avec un monoisocyanate d'alkyle ;
  Par exemple, les monoisocyanates peuvent êtres choisis parmi l'isocyanate de butyle, d'isobutyle, de pentyle, d'hexyle, d'heptyle, d'octyle, de 2-éthylhexyle, de nonyle, de décyle, d'undécyle, de dodécyle.

- réaction avec un monoalcoxysilane ;
  Par exemple, les monoalcoxysilanes peuvent êtres choisis parmi le diméthylméthoxybutylsilane, le diméthylméthoxypentylsilane, le diméthylméthoxy-hexylsilane, le diméthylméthoxy(alkyl en $C_7$ à $C_{12}$) silane ;

- réaction avec un monoépoxyde, catalysée en milieu acide ou basique ou en présence d'alumine.
  Par exemple, le monoépoxyde peut être choisi parmi le 1,2-époxyhexane, le 1,2-époxyheptane, le 1,2-époxyoctane, le 1,2-époxynonane, le 1,2-époxynéodécane (commercialisé par Shell sous la marque Cardura. E)

- réaction avec un halogénure d'alkyle ;
  Par exemple, l'halogénure d'alkyle peut être choisi parmi les chlorures d'alkyle tels que le 1-chlorobutane, le 1-chloropentane, le 1-chlorohexane.

- réaction avec un monoacétal d'alkyle en $C_1$-$C_4$ en milieu acide ;
  Par exemple, on peut citer comme monoacétals d'alkyle le butylméthyléther, le pentylméthyléther, l'hexylméthyléther, le dodécylméthyléther.

- réaction d'addition des fonctions -OH de la nitrocellulose sur un réactif porteur d'une double liaison.

Du point de vue des conditions opératoires, la réaction de greffage, à partir des réactions et réactifs spécifiés ci-dessus, sera réalisée de préférence, par mise en solution de la nitrocellulose et des réactifs de greffage dans un solvant ou mélange de solvants dépourvu d'oxygène. La concentration en nitrocellulose dans la solution est, par exemple, de 0,5 % à 30 %, de préférence de 1 à 10 %. La température de réaction est portée de 0 à 80°C, suivant la réaction et le réactif choisi. Suivant le type de réactions, on peut ajouter un ou plusieurs catalyseurs. L'avancement de la réaction est suivi par des techniques classiques telles que les spectroscopies IR, RMN, UV ou la chromatographie sur couche mince (CCM). Le produit obtenu est ensuite purifié et analysé par des techniques classiques de purification et d'analyse.
Selon un second mode de réalisation du procédé selon l'invention, correspondant au cas où la chaîne hydrocarbonée R est d'origine polymérique, la réaction de greffage de ladite chaîne peut être effectuée de différentes façons.
Ainsi, selon une première réalisation, la réaction de greffage peut consister à faire réagir directement sur tout ou partie des fonctions hydroxyles libres de la nitrocellulose de départ, les extrémités réactives de polymères, dont les chaînes constituent en fait la chaîne hydrocarbonée R.
La réaction de greffage peut être représentée par la réaction globale suivante :

$$\text{Nitrocell-OH} + X_1\text{-POL} \rightarrow \text{Nitrocell-O-Y-POL}$$

avec POL représentant le polymère dont la séquence répond à la définition de la chaîne hydrocarbonée R donnée précédemment, $X_1$ représentant une fonction portée par le polymère, ladite fonction étant réactive vis-à-vis des hydroxyles de la nitrocellulose et Y répondant à la même définition que celle donnée précédemment et résultant de la réaction de -OH avec $X_1$.
Au même titre que pour le greffage de chaînes hydrocarbonées de nature non polymérique, les fonctions réactives $X_1$ vis-à-vis des fonctions hydroxyles libres de la nitrocellulose, peuvent être choisies parmi les fonctions époxyde, aldéhyde, acétal, halogène (chlore, brome, iode), éthylénique, acide carboxylique ou dérivé (chlorure, anhydride, ester d'alkyle en $C_1$-$C_4$), carbonate, acide sulfonique ou chlorure de sulfonyle, isocyanate, monoalcoxysilane.
Toutefois, compte tenu des dangers de la manipulation de la nitrocellulose mentionnée ci-dessus, les fonctions réactives $X_1$ des polymères hydrocarbonées, engagées dans la réaction de greffage, sont de préférence, choisies parmi les fonctions chlorure d'acide carboxylique, anhydride d'acide carboxylique, acide carboxylique (en présence d'un réactif de couplage du type DCCI), monoisocyanate, monoalcoxysilane, monoépoxyde, halogène (Cl, Br, I), monoéther d'alkyle $C_1$-$C_4$, double liaison vinylique.
Les polymères POL-$X_1$ de départ nécessitent d'être synthétisés, mis à part ceux pour lesquels $X_1$ est une

double liaison réactive type vinylique, dont de nombreux sont disponibles dans le commerce. Les polymères POL-$X_1$ peuvent être synthétisés par exemple à partir d'un polymère comportant une fonction réactive différente de $X_1$ que l'on transforme par des réactions classiques en $X_1$ appropriée.

A titre d'exemples, on peut citer la réaction suivante :

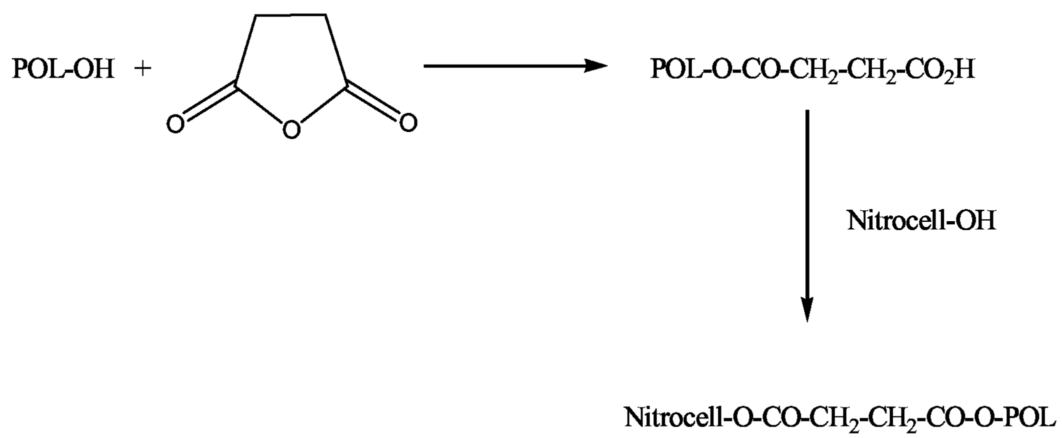


Selon une deuxième réalisation, la réaction de greffage peut consister, dans un premier temps, à transformer tout ou partie des fonctions hydroxyles de la nitrocellulose de départ en fonctions réactives, puis, dans un deuxième temps, à faire réagir lesdites fonctions réactives avec les extrémités réactives adéquates de polymères comportant ladite chaîne hydrocarbonée R.

A titre d'exemples, on peut citer la réaction suivante :

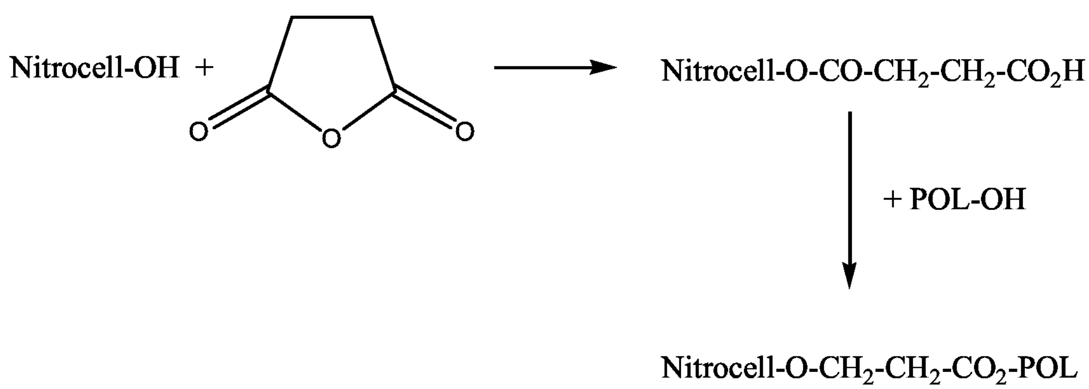


La réaction Nitrocell-$OCOCH_2CH_2$-$CO_2H$ avec Nitrocell-OH peut avoir lieu mais on se met dans des conditions, où cette réaction est minimisée (on évite la réticulation).

Concernant la nature du polymère pouvant être engagé dans la réaction de greffage avec la nitrocellulose, pour constituer les chaînes hydrocarbonées R, celui-ci peut être de toute nature chimique (polymères vinyliques, polycondensats tels que des polyesters, des polyamides, des polyuréthanes, polyurée..etc), à condition que ledit polymère satisfasse à la définition de la chaîne hydrocarbonée R définie ci-dessus.

De tels polymères peuvent être choisis, par exemple, parmi les polymères vinyliques et les polycondensats.

Selon l'invention, les polymères vinyliques désignent, aussi bien les oligomères que les polymères issus de l'homopolymérisation ou la copolymérisation de monomères vinyliques au sens large, c'est-à-dire de monomères (méth)acryliques, styréniques, allyliques.

A titre d'exemples de polymères vinyliques, on peut citer les polymères obtenus par homopolymérisation ou copolymérisation des monomères choisis parmi :

- les acrylates d'alkyle avec un groupe alkyle en $C_1$-$C_{30}$, linéaire ou ramifié, tels que l'acrylate de méthyle, d'éthyle, de propyle, d'isopropyle, de n-butyle, d'isobutyle, de n-pentyle, de n-hexyle, de n-heptyle, de n-octyle, de 2-éthylhexyle, de nonyle, de décyle, d'undécyle, de dodécyle.

- les méthacrylates d'alkyle avec un groupe alkyle en $C_6$-$C_{30}$ ou un groupe alkyle en $C_1$-$C_5$, dans le cas de copolymères, à condition que la température de transition vitreuse satisfasse à la condition mentionnée ci-après.
- les esters vinyliques, avec un groupe alkyle en $C_3$-$C_{30}$, linéaire ou ramifié tel que le propionate de vinyle, le néodécanoate de vinyle, l'octanoate de vinyle, le décanoate de vinyle, le laurate de vinyle ou un groupe alkyle en $C_1$-$C_2$, dans le cas de copolymères, à condition que la température de transition vitreuse dudit copolymère satisfasse à la condition mentionnée ci-après.
- les éthers vinyliques avec un groupe alkyle en $C_3$-$C_{30}$ tels que le propylvinyléther, le butylvinyléther, l'isobutylvinyléther le tertiobutylvinyléther, le pentylvinyléther, l'hexylvinyléther l'octylvinyléther le 2-éthylhexylvinyléther, le nonylvinyléther, le décylvinyléther, le dodécylvinyléther ;
- les $\alpha$-oléfines, par exemple en $C_2$ à $C_{20}$, en particulier les copolymères d'$\alpha$-oléfines, dont les monomères donnent des homopolymères cristallins ; et des homopolymères ou copolymères d'$\alpha$-oléfines ramifiées. Citons en particulier l'homopolymère d'isobutylène et les copolymères entre éthylène (ou propylène) et d'$\alpha$-oléfines plus longues telles que le butène, l'hexène, l'octène, le décène, le dodécène. Pour ces $\alpha$-oléfines, on peut également citer leurs copolymères non cristallins avec les cyclooléfines, en particulier, les copolymères entre éthylène (ou propylène) et norbornène ou les dérivés du norbornène ;
- les diènes, par exemple, en $C_4$ à $C_{20}$, tels que le butadiène, l'isoprène, l'hexadiène, ...etc donnant des copolymères avec d'autres monomères vinyliques tel que ceux mentionnés ci-dessus, et en plus avec le styrène ou styrènes substitués.

Comme indiqué précédemment, les chaînes hydrocarbonées R greffées au niveau de la nitrocellulose, peuvent comporter un ou plusieurs groupes promoteurs d'adhérence. Ces groupes promoteurs d'adhérence peuvent être introduits par copolymérisation avec des monomères vinyliques appropriés, tels que :

- des monomères porteurs de groupes $-CO_2H$, tels que l'acide (méth)acrylique, l'acide crotonique, l'acide maléique, l'acide itaconique, l'acide allyloxyacétique ;
- des monomères porteurs de groupes -OH, tels que l'alcool allylique, le (méth)acrylate de 2-hydroxyéthyle, de 2-hydroxypropyle ;
- des monomères porteurs de groupes amides tels que le (méth)acrylamide, le N-alkyl (méth)acrylamide avec des alkyls en $C_1$-$C_{12}$ égaux ou différents, le N-vinylacétamide, le N-vinylformamide ;
- des monomères porteurs de groupes pyrrolidono tels que la N-vinylpyrrolidone ;
- des monomères porteurs de groupes amines tels que la vinylamine, l'allylamine, le (méth)acrylate de N,N-diméthylaminoéthyle ;
- des monomères porteurs de groupes oxazolines, tels que la N-vinyloxazoline ;
- des monomères porteurs de groupes pyridine tels que la N-vinylpyridine ;
- des monomères porteurs de groupes chlore, tels que le chlorure de vinyle, de chlorure de vinylidène, les chlorooléfines ;
- des monomères porteurs de groupes acétoacétyle tels que le 2-acétoacétoxy(méth)acrylate ;
- des monomères porteurs de groupes acétals tels que le (méth)acrylamidobutyraldéhydediméthylacétal, le (méth) acrylamidoacétaldéhydediméthylacétal ;
- des monomères porteurs de groupes siloxanes tels que le 3-(triméthoxysilyl)-propylméthacrylate.

A titre d'exemples de polycondensats, on peut citer :

- les polyesters tels que ceux issus de la polyestérification entre les diols et diacides tels que l'éthylène glycol, le propylène glycol, le diéthylène glycol, le néopentyl glycol, le 1,4-butanediol, le furanne diméthanol, le cyclohexane diméthanol, le glycérol, le triméthylolpropane, le pentaérythitol et leurs mélanges, avec des acides polycarboxyliques, en particulier des acides carboxyliques et leurs dérivés esters en $C_1$-$C_4$, par exemple, l'acide succinique, glutarique et adipique ou leurs esters de diméthyle, l'anhydride phtalique ou le téréphtalate de diméthyle ou avec des lactones, par exemple la caprolactone ;
- les polyesteramides obtenus par inclusion d'aminoalcools tels que l'éthanolamine dans des mélanges de polyestérification ;
- les polycarbonates, obtenus par réaction de diols tels que le 1,3-propanediol, le 1,4-butanediol, le 1,8-hexanediol, le diéthylèneglycol, le tétraéthylèneglycol avec des carbonates de diaryle, de diacyle, ou aliphatiques par exemple le carbonate de diphényle, ou avec du phosgène ;
- les polyamides obtenus par condensation entre un diacide carboxylique (ou dérivé ester en $C_1$-$C_4$) aliphatique, cycloaliphatique ou aromatique en $C_3$-$C_{50}$ et une diamine en $C_2$-$C_{50}$ aliphatique, linéaire ou ramifiée, cycloaliphatique ou aromatique, les diacides pouvant être choisis parmi les diacides mentionnés ci-dessus, les diamines pouvant être choisis parmi l'éthylènediamine le 1,2-diaminopropane, le 1,3-diaminopropane, le 1,4-diaminobutane,

le 1,2-diamino-2-méthylpropane, le 1,6-diaminohexane, le 1,10-diaminodécane,l'isophoronediamine, l'adamantanediamine la 2,6-diaminopyridine ;

- les polyuréthanes, polyurées et polyurées-uréthanes obtenus par polyaddition entre des diisocyanates aliphatiques, cycloaliphatiques et/ou aromatiques de $C_4$-$C_{100}$, de préférence $C_4$-$C_{30}$ tels que l'hexaméthylène diisocyanate, l'isophorone diisocyanate, le toluène diisocyanate, le diphénylméthane diisocyanate et des diols tels que définis ci-dessus, ou des diamines telles que définies ci-dessus, ou des mélanges diols/diamines ;
- les polyéthers tels que les copolymères entre oxyéthylène et oxypropylène, le polytétraméthylèneoxyde.

En ce qui concerne l'introduction de groupes promoteurs d'adhérence dans ce type de polycondensats, ces groupes sont introduits directement par le biais de monomères incluant ces groupes.

A titre d'exemples non limitatifs, pour l'introduction de groupes $-CO_2H$, on peut utiliser l'acide diméthylolpropionique (en particulier pour les polyuréthanes, les polyurées) ; pour l'introduction de groupes amino, on peut utiliser la N,N-diméthyléthanolamine (en particulier pour les polyurées).

Les polymères, ainsi que les polycondensats présentant un groupe réactif vis-à-vis des fonctions hydroxyles libres de la nitrocellulose, peuvent être préparés de différentes façons.

Ainsi, pour les polymères porteurs d'un groupe réactif, une première variante consiste à préparer lesdits polymères par polymérisation du ou des monomères choisis en présence d'un agent de transfert porteur d'un groupe réactif.

Le polymère ainsi formé comporte deux extrémités, soit une extrémité $\alpha$ comportant un reste d'amorceur de polymérisation et une extrémité $\omega$ comportant le groupe provenant de l'agent de transfert.

Les conditions de polymérisation, le choix de l'agent de transfert sont, de préférence, telles que la réaction de terminaison des chaînes se fait par transfert.

Par exemple, pour préparer un télomère de poly(acrylate d'éthyl-2-hexyle) à terminaison réactive et de poids moléculaire 1150, on peut procéder en deux étapes telles que cela est décrit dans la demande de brevet international WO-A-97/35541.

Dans une première étape, l'on polymérise l'acrylate d'éthyl-2-hexyle par amorçage avec l'azobisisobutyronitrile avec le mercaptoéthanol comme agent de transfert, afin d'obtenir un polymère avec une extrémité -OH.

Dans une deuxième étape, l'on transforme l'extrémité -OH non réactive vis-à-vis des OH- libres de la nitrocellulose, en groupe réactif, par exemple, par réaction avec un anhydride cyclique, tel que l'anhydride succinique, dans des conditions telles que la double estérification ne puisse avoir lieu.

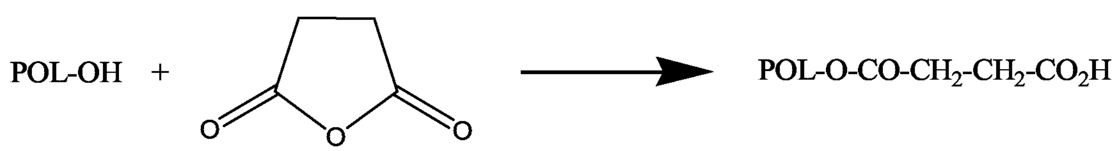

Pour obtenir directement un groupe réactif $-CO_2H$, on peut utiliser comme agent de transfert, l'acide mercaptopropionique, afin d'obtenir le polymère à extrémité réactive POL-S-$CH_2$-$CH_2$-$CO_2H$.

D'autres polymères terminés par une extrémité vinylique (au sens large dont (méth)acrylique, styrénique, allylique) existent dans le commerce et peuvent, de ce fait, être utilisés directement dans la réaction de greffage à la nitrocellulose. De tels oligomères sont, en particulier, commercialisés par la société japonaise TOA GOSEI.

En ce qui concerne les polycondensats à extrémité réactive $X_1$, leur préparation, notamment en ce qui concerne les polyesters et les polyamides, ne nécessite pas d'aménagement particulier pour l'introduction du groupe réactif, dans la mesure où celui-ci existe déjà en extrémité de chaîne.

Par exemple, un polyester comporte, en général, en fin de préparation une extrémité $-CO_2H$ réactive et une extrémité -OH. Il est à noter que cette extrémité -OH sera, de préférence, bloquée par un groupe inerte dépourvu d'hydrogène labile, afin de ne pas gêner la réaction de greffage sur la nitrocellulose.

Les mêmes remarques sont applicables pour un polyamide, qui présente une extrémité $-CO_2H$ réactive et une extrémité $-NH_2$ à protéger par un groupe inerte vis-à-vis de la réaction de greffage avec la nitrocellulose.

On peut introduire, également un groupe réactif $X_1$ au niveau du polycondensat, par introduction dans le milieu réactionnel, en cours de polycondensation, d'un réactif porteur du groupe $X_1$, qui doit être inerte vis-à-vis du type de polycondensation choisi ou inerte dans les conditions expérimentales de la polycondensation, et d'un seul groupe apte à participer à la polycondensation. Ce réactif est donc monofonctionnel vis-à-vis de la polycondensation et sert donc de limiteur de chaîne.

Ce réactif monofonctionnel vis-à-vis de la polycondensation et porteur d'un groupe réactif vis-à-vis des fonctions hydroxyles de la nitrocellulose, est, de préférence, introduit au cours de la polycondensation, pour que les chaînes du polymère ne soient terminées que par un seul groupe réactif $X_1$.

Selon une troisième réalisation de greffage de chaînes hydrocarbonées R de nature polymérique sur les fonctions hydroxyles libres d'une nitrocellulose, la réaction de greffage consiste, dans un premier temps, à transformer tout ou partie des fonctions hydroxyles libres de la nitrocellulose de départ en

fonctions aptes à remplir le rôle de co-amorceur d'une réaction de polymérisation vinylique puis dans un deuxième temps, à faire réagir ladite nitrocellulose transformée avec des monomères vinyliques.

Cette réalisation peut être mise en oeuvre, en faisant appel notamment, aux nouveaux types de polymérisation radicalaire contrôlée dite « vivante », tel que le procédé d' « Atom Transfer Polymerization » (ATRP). Ce procédé nécessite, au préalable, de transformer les -OH libres de la nitrocellulose en groupes porteurs d'un atome d'halogène (chlore, brome), lesdits groupes servant de co-amorceurs et sites de greffage pour une polymérisation ATRP vinylique amorcée avec un système sel de Cu(I) ou sel de Cu(I)/bipyridine, tel que cité dans les demandes de brevets WO 97/18247 et WO 98/51722.

Un tel procédé de modification des -OH puis amorçage d'une polymérisation ATRP en présence de Cu(I) est décrit dans les demandes de brevet WO 071 606 et WO 071 607 et dans l'article de MM.Guerrini, Macromol.Rapid. Commun., 21, N°10, pages 1-6 (2000).

Ainsi, le processus de greffage de la nitrocellulose, selon un procédé ATRP, est alors le suivant :

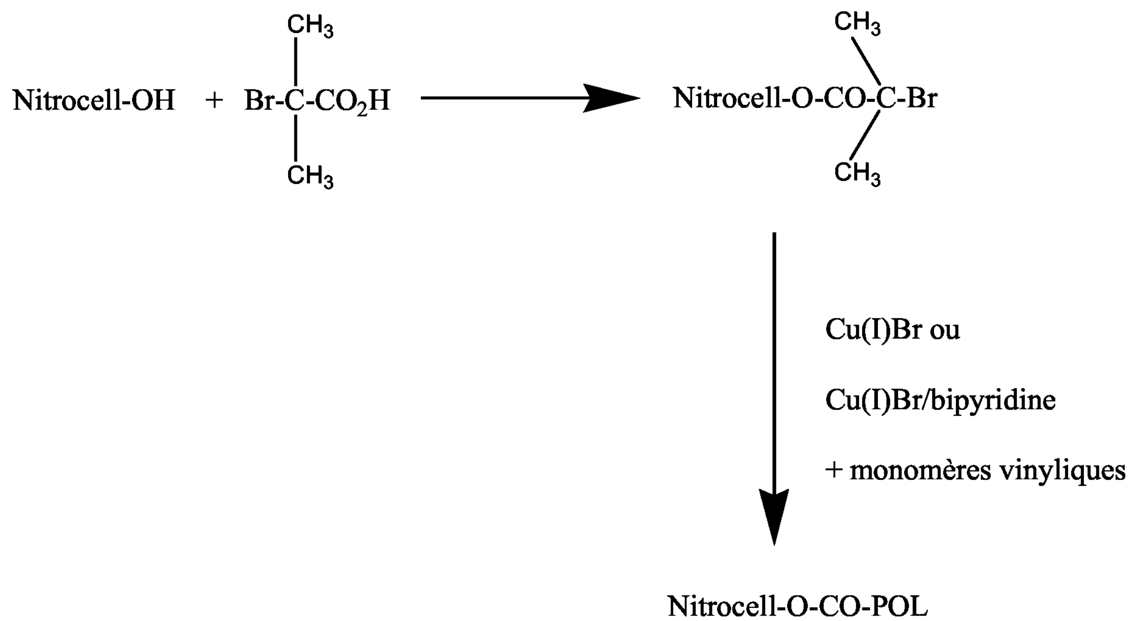


POL représentant le polymère issu de la polymérisation des monomères vinyliques, tels que les (méth)acrylates d'alkyle.

De préférence, les polymères, utilisables comme greffons de la nitrocellulose, selon l'invention, présentent, une température de transition vitreuse inférieure ou égale à 60 °C, notamment allant de -150°C à 60°C, de préférence inférieure ou égale à 40 °C, notamment allant de -150°C à 40°C, ou une température de fusion inférieure ou égale à 60 °C, de préférence inférieure ou égale de 40°C. Les valeurs de température de transition vitreuse ou de fusion peuvent être déterminées de façon connue, par les valeurs de température de transition vitreuse ou de fusion des monomères, qui sont, par exemple, celles que l'on peut trouver dans un manuel de référence tel que le Polymer Handbook, 3$^{rd}$ ed, 1989, John Wiley.

Enfin, en alternative, les nitrocelluloses modifiées conformes à la présente invention peuvent être préparées en effectuant une réaction de greffage de chaînes R, telle que définie précédemment, sur une partie des fonctions -OH libres d'une cellulose de départ pour obtenir une cellulose dont une partie des-OH ont été remplacés par des groupes -O-Y-R tels que définis ci-dessus, suivie d'une nitration de tout ou partie des fonctions -OH restantes, de manière à obtenir la nitrocellulose modifiée souhaitée.

L'invention va maintenant être décrite en référence aux exemples suivants donnés à titre illustratif et non limitatif.

## DESCRIPTION DÉTAILLÉE DE MODES DE RÉALISATION PARTICULIERS.

Les exemples suivants illustrent la préparation de nitrocelluloses modifiées, selon la présente invention, ainsi que des exemples de formulation, comprenant de tels nitrocelluloses.

### Exemple 1 : Préparation d'une nitrocellulose modifiée selon l'invention.

Dans cet exemple, on prépare une nitrocellulose modifiée, comportant des radicaux -OYR, remplaçant en partie, les hydroxyles libres de la nitrocellulose de départ, Y étant un groupe de liaison -(C=O)-NH- et R une chaîne-$(CH_2)_7$-$CH_3$.

Dans un tricol surmonté d'un réfrigérant, 122 g de nitrocellulose (Référence NCE20 de Bergerac, contenant 18% en poids de phtalate de dibutyle) est dissous dans 1 litre d'un mélange de méthyléthylcétone-toluène (60/40 v/v) anhydre, sous agitation. La solution est dégazée trois fois et mise sous argon. Toujours sous agitation, et en présence d'argon, 24,75 g d'octylisocyanate est ajouté dans le milieu réactionnel. Après addition d'une quantité catalytique de dibutylétain dilaurate, la solution est chauffée à 50 °C et maintenue à cette température pendant 48 heures. Après retour à température ambiante, le milieu réactionnel est mis en présence de 5 litres d'eau, où la nitrocellulose modifiée précipite. Celle-ci est récupérée et lavée 3 fois avec un grand excès d'eau. Elle est ensuite contre-dialysée dans de l'acétone pendant 5 jours, afin d'éliminer les impuretés et le phtalate de dibutyle initialement présent dans la nitrocellulose. A l'issue de la dialyse, l'acétone est évaporée de façon contrôlée jusqu'à ce que la quantité finale corresponde à une composition de 50% en acétone pour 50% en polymère.

1 g de la solution obtenue est séchée à 50°C, jusqu'à l'évaporation complète du solvant. Le film de polymère ainsi obtenu est analysé en infrarouge. Il y a apparition d'une bande à 2255 $cm^{-1}$, correspondant à la présence de la liaison carbamate entre les fonctions hydroxy de la nitrocellulose et la fonction isocyanate de l'octylisocyanate.

Le polymère ainsi obtenu présente une composition finale (en poids) de 65 % de motifs modifiés pour 35 % de motifs non modifiés.

### <u>Exemple 2 :</u>

On prépare un vernis à ongles colorés ayant la composition suivante :

- nitrocellulose modifiée de l'exemple 1: 20 g
- plastifiant : 0 à 20g
- polymère filmogène additionnel : 0 à 20 g
- gélifiant : 0 à 10 g
- alcool : 0 à 15 g
- matières colorantes : 0 à 10 g
- solvants (mélange acétate de butyle, acétate d'éthyle) : qsp 100 g.

Les taux de plastifiants et de polymère filmogènes additionnels sont adaptés aux critères mécaniques du film pré définis.

Le vernis à ongles s'applique bien sur ongles, le film obtenu est brillant et offre une bonne tenue après 5 jours sans usure ni écaillage.

### <u>Exemple 3 :</u>

On prépare un vernis à ongles ayant la composition suivante :

- nitrocellulose modifiée de l'exemple 1 : 20 g
- N-éthyl, o,p-toluène sulfonamide : 10 g
- Hectorite : 1,7 g

- Pigment rouge DC Red 34 : 1,5 g
- Solvants (acétate d'éthyle, acétate de butyle) : qsp 100 g

Le vernis s'applique facilement sur l'ongle. Le film de maquillage adhère bien sur l'ongle et présente une bonne tenue, notamment une bonne résistance à l'écaillage.

**<u>Exemple 4 :</u>**

On prépare un vernis à ongles ayant la composition suivante :

- nitrocellulose modifiée de l'exemple 1 : 20 g
- Acétyl tributyl citrate: 8 g
- Hectorite: 1,7 g
- Pigment rouge DC Red 34: 1,5 g
- Solvants (acétate d'éthyle, acétate de butyle) : qsp 100 g

Le vernis s'applique facilement sur l'ongle et le maquillage obtenu est brillant, adhère bien sur l'ongle et présente une bonne résistance à l'écaillage.

**Revendications**

1. Composition cosmétique comprenant au moins un solvant organique et une nitrocellulose comportant des fonctions hydroxyles libres remplacées, en tout ou partie, par des radicaux -OYR, où R représente une chaîne hydrocarbonée linéaire ou ramifiée comportant de 1 à 500 atomes de carbone, ou cyclique comportant de 3 à 500 atomes de carbone, ladite chaîne étant saturée ou insaturée et pouvant comporter un ou plusieurs atomes de O, N, S, Si et/ou P, et Y représente une liaison simple ou un groupe de liaison.

2. Composition cosmétique selon la revendication 1, dans laquelle les chaînes hydrocarbonées R sont liées sur les atomes d'oxygène des cycles anhydroglucose de la nitrocellulose par des groupes de liaisons Y choisis parmi les groupes -(C=O)-, -(C=O)O-, $-SO_2$, -CO-NH- ou -CO-NR' -, -Si $(R_3)_2$-, les $R_3$ identiques ou différents, étant une chaîne hydrocarbonée linéaire ou ramifiée de 1 à 500 atomes de carbone, ou cyclique de 3 à 500 atomes de carbone, ladite chaîne étant saturée ou insaturée et pouvant comporter un ou plusieurs atomes de O, N, S, Si et/ou P, et R' désignant un radical alkyl en C1 à C4.

3. Composition cosmétique selon la revendication 1 ou 2, dans laquelle les chaînes hydrocarbonées R ont de 4 à 100 atomes de carbone.

4. Composition cosmétique selon l'une quelconque des revendications 1 à 3, dans laquelle les chaînes hydrocarbonées R comportent au moins un groupe promoteur d'adhérence.

5. Composition cosmétique selon l'une quelconque des revendications 1 à 4, dans laquelle les groupes promoteurs d'adhérence sont situés dans les chaînes hydrocarbonées R latéralement et/ou à leur extrémité.

6. Composition cosmétique selon la revendication 4 ou 5, dans laquelle les groupes promoteurs d'adhérence sont présents à raison d'une teneur inférieure ou égale à 10 % en poids de la nitrocellulose, de préférence inférieure ou égale à 5 %.

7. Composition cosmétique selon l'une quelconque des revendications 4 à 6, dans laquelle les groupes promoteurs d'adhérence sont choisis parmi les groupes suivants :

   - hydroxyle -OH ;
   - acide carboxylique ou ester $-CO_2R_1$;

- chloro -Cl ;
- amino du type $-NR_1R_2$ ;
- pyridino de formule :

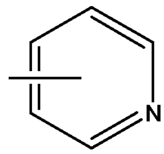

- pyrimidino de formule :

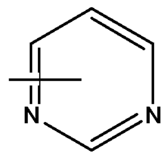

- oxazolino de formules :

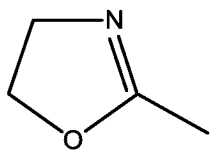
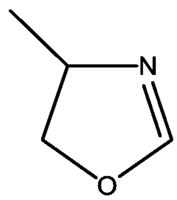
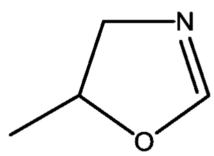

- amido -NH-CO-R' ou $-CO-NH-R_1$ ;
- pyrrolidono de formules :

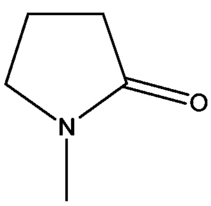
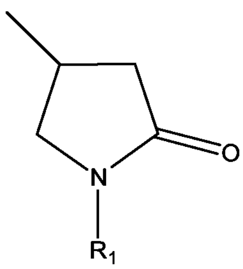
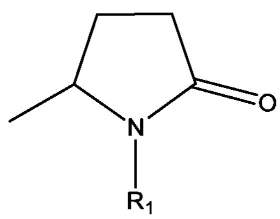
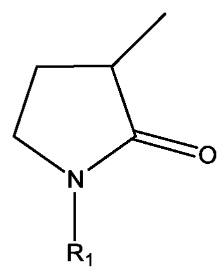

- carbamoyl -O-CO-NH-R' ou -NH-CO-O-R' ;
- thiocarbamoyl de formule $-O-CS-NHR_1$ ou -NH-CS-O-R' ;
- carbonato -O-CO-O-R' ;
- uréyl du type $-NR_1-CO-N\ (R_1)\ _2$, les $R_1$ étant identiques ou différents ;
- thiouréyl $-NR_1-CS-N\ (R_1)_2$, les $R_1$ étant identiques ou différents ;
- oxamido $-NR_{1-}CO-CO-N\ (R_1)\ _2$ avec les $R_1$ identiques ou différents ;
- guanidino $-NH-C(=NH)-N(R_1)_2$ avec les $R_1$ identiques ou différents ;
- biguanidino $-NH-C(=NH)-NH-C(=NH)-N(R_1)_2$ avec les $R_1$ identiques ou différents ;
- ester sulfonique $-O-S(=O)_2-R'$;
- sulfonamido $-NR_1-S(=O)_2-R'$ ;
- acétoacétyl $-O-CO-(CH_2)_a-CO-R'$ avec a désignant un nombre entier allant de 1 à 10, de préférence allant de 1 à 5 ;
- siloxane $-Si-(R')\ _{3-b}\ (OR')_b$ avec b désignant un nombre entier allant de 1 à 3 ;

- acétal -OR',
  les $R_1$ et $R_2$ désignant H ou un radical alkyle en C1 à C4 et R' désignant un radical alkyle en C1 à C4.

**8.** Composition cosmétique selon l'une quelconque des revendications 1 à 7, dans laquelle le taux de greffage molaire des radicaux de formule OYR remplaçant, en tout ou partie, les groupes hydroxyles libres par cycle anhydroglucose de la nitrocellulose, est de 0,1 à 2, préférentiellement de 0,25 à 0,80.

**9.** Composition cosmétique selon l'une quelconque des revendications précédentes, dans laquelle les chaînes hydrocarbonées R sont liées sur les atomes d'oxygène des cycles anhydroglucose de la nitrocellulose par des liaisons simples.

**10.** Composition cosmétique selon l'une quelconque des revendications précédentes, dans laquelle les chaînes hydrocarbonées sont de nature polymérique et/ou non polymérique.

**11.** Composition cosmétique selon la revendication 10, dans laquelle les chaînes, lorsqu'elles sont de nature polymérique, ne sont pas des chaînes vinyliques et/ou acryliques.

**12.** Composition cosmétique selon l'une quelconque des revendications précédentes, dans laquelle la nitrocellulose présente une seule température de transition vitreuse Tg telle que Tg soit inférieure ou égale à 100 °C, de préférence allant de 30 à 70°C, et mieux encore de 40 à 60°C.

**13.** Composition cosmétique selon l'une quelconque des revendications 1 à 12, dans laquelle la nitrocellulose est apte à former un film ayant un module de conservation E' supérieur ou égal à 100 Mpa, de préférence supérieur ou égal à 300 MPa et/ou un pouvoir amortissant tg$\delta$ supérieur ou égal à 0,4, de préférence supérieur ou égal à 0,6, à une température de 30 °C et à une fréquence de 1 Hz.

**14.** Composition cosmétique selon l'une quelconque des revendications 1 à 13, dans laquelle la nitrocellulose est apte à former un film ayant une déformation à la rupture $\varepsilon_r$ supérieure ou égale à 5 %, de préférence supérieure ou égale à 15% et/ou une énergie à rupture par unité de volume $W_r$ supérieure ou égale à 0,2 J/$cm^3$, de préférence supérieure ou égale à 1J/$cm^3$ .

**15.** Composition cosmétique selon l'une quelconque des revendications 1 à 14, ladite composition étant une composition de vernis à ongles destinée au maquillage des ongles et/ou faux-ongles.

**16.** Composition cosmétique selon l'une quelconque des revendications 1 à 15, dans laquelle la teneur en nitrocellulose modifiée est de 0,1 à 60% en poids, de préférence de 1 à 50% en poids, et mieux encore de 5 à 40% en poids, par rapport au poids total de la composition.

**17.** Composition cosmétique selon l'une quelconque des revendications 1 à 16, dans laquelle le solvant organique est choisi parmi les cétones liquides à température ambiante, les alcools liquides à température ambiante, les glycols liquides à température ambiante, les éthers de propylène glycol liquides à température ambiante, les esters comportant de 3 à 8 atomes de carbone au total, les éthers liquides à température ambiante, les alcanes liquides à température ambiante, les composés cycliques aromatiques liquides à température ambiante, les aldéhydes liquides à température ambiante et les mélanges de ceux-ci.

**18.** Composition cosmétique selon l'une quelconque des revendications 1 à 17, comprenant, en outre, un ou plusieurs additifs choisis parmi les polymères filmogènes additionnels, les agents plastifiants, les matières colorantes telles que les pigments, les nacres, les paillettes, les agents épaississants, les agents d'étalement, les agents mouillants, les agents dispersants, les anti-mousses, les conservateurs, les filtres UV, les actifs, les tensioactifs, les cires, les agents hydratants, les parfums, les neutralisants, les stabilisants, les antioxydants.

**19.** Nitrocellulose comportant des fonctions hydroxyles libres remplacées, en tout ou partie, par des radicaux -OYR, où R représente une chaîne hydrocarbonée linéaire, ramifiée comportant de 1 à 500 atomes de carbone, ou cyclique comportant de 3 à 500 atomes de carbone, ladite chaîne étant saturée ou insaturée et pouvant comporter un ou plusieurs atomes de O, N, S, Si et/ou P, et Y représente une liaison simple ou un groupe de liaison et ladite chaîne comprenant au moins un groupe promoteur d'adhérence choisi parmi les groupes suivants :

- chloro -Cl ;

- amino $-NR_1R_2$ avec les $R_1$ et $R_2$, identiques ou différents ;
- pyridino de formule :

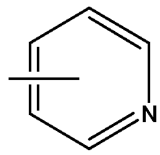

- pyrimidino de formule :

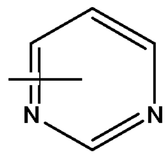

- oxazolino de formules :

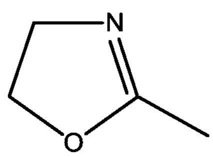
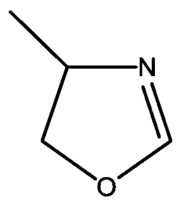
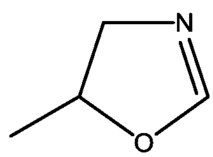

- amido -NH-CO-R' ou $-CO-NH-R_1$ ;
- pyrrolidono de formules :

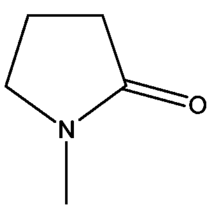
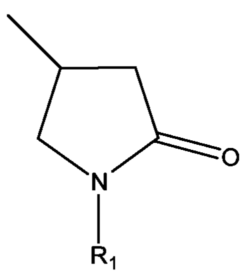
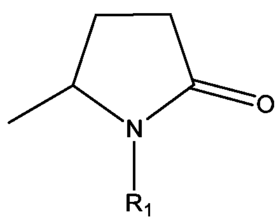
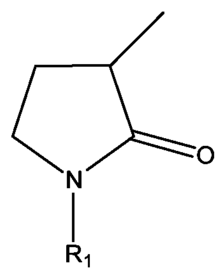

- carbamoyl -O-CO-NH-R' ou -NH-CO-O-R';
- thiocarbamoyl de formule $-O-CS-NHR_1$ ou -NH-CS-O-R' ;
- carbonato -O-CO-O-R';
- uréyl $-NR_1-CO-N(R_1)_2$, les $R_1$ étant identiques ou différents ;
- thiouréyl $-NR_1-CS-N(R_1)_2$, les $R_1$ étant identiques ou différents ;
- oxamido $-NR_1-CO-CO-N(R_1)_2$ avec les $R_1$ identiques ou différents ;
- guanidino $-NH-C(=NH)-N(R_1)_2$ avec les $R_1$ identiques ou différents ;
- biguanidino $-NH-C(=NH)-NH-C(=NH)-N(R_1)_2$ avec les $R_1$ identiques ou différents ;
- ester sulfonique $-O-S(=O)_2-R'$ ;
- sulfonamido $-NR_1-S(=O)_2-R'$ ;
- acétoacétyl $-O-CO-(CH_2)_a-CO-R'$ avec a désignant un nombre entier allant de 1 à 10, de préférence allant de 1 à 5 ;
- siloxane $-Si-(R')_{3-b}(OR')_b$ avec b désignant un nombre entier allant de 1 à 3 ;
- acétal -OR',

les $R_1$ et $R_2$ désignant H ou un radical alkyle en C1 à C4 et R' désignant un radical alkyle en C1 à C4.

**20.** Nitrocellulose selon la revendication 19, dans laquelle les chaînes hydrocarbonées R sont liées sur les atomes d'oxygène des cycles anhydroglucose de la nitrocellulose par des groupes de liaisons Y choisis parmi les groupes -(C=O)-, -(C=O)O-, $-SO_2$, -CO-NH- ou -CO-NR'-,$-Si(R_3)_2-$, les $R_3$ identiques ou différents, étant une chaîne hydrocarbonée linéaire, ramifiée ou cyclique de 1 à 500 atomes de carbone, saturée ou insaturée, pouvant comporter un ou plusieurs atomes de O, N, S, Si et/ou P, $R_3$ comportant, de préférence, de 1 à 10 atomes de carbone et R' représentant un groupe alkyle en C1 à C4.

**21.** Nitrocellulose selon la revendication 19 ou 20, dans laquelle les chaînes hydrocarbonées R ont de 8 à 100 atomes de carbone.

**22.** Nitrocellulose selon l'une quelconque des revendications 19 à 21, dans laquelle les groupes promoteurs d'adhérence sont situés dans les chaînes hydrocarbonées R latéralement et/ou à leur extrémité.

**23.** Nitrocellulose selon l'une quelconque des revendications 19 à 22, dans laquelle les groupes promoteurs d'adhérence sont présents à raison d'une teneur inférieure ou égale à 10 % en poids de la nitrocellulose, de préférence inférieure ou égale à 5 %.

**24.** Nitrocellulose selon l'une quelconque des revendications 19 à 23, dans laquelle le taux de greffage molaire des radicaux de formule OYR remplaçant, en tout ou partie, les groupes hydroxyles libres par cycle anhydroglucose de la nitrocellulose, est de 0,1 à 2, préférentiellement de 0,25 à 0,80.

**25.** Nitrocellulose selon l'une quelconque des revendications 19 à 24, dans laquelle les chaînes hydrocarbonées R sont liées sur les atomes d'oxygène des cycles anhydroglucose de la nitrocellulose par des liaisons simples.

**26.** Nitrocellulose selon l'une quelconque des revendications précédentes, dans laquelle les chaînes hydrocarbonées sont de nature polymérique et/ou non polymérique.

**27.** Nitrocellulose selon l'une quelconque des revendications précédentes, présentant une seule température de transition vitreuse Tg telle que Tg soit inférieure ou égale à 100 °C, de préférence allant de 30 à 70°C, et mieux encore de 40 à 60°C.

**28.** Nitrocellulose selon l'une quelconque des revendications 19 à 27, apte à former un film ayant un module de conservation E' supérieur ou égal à 100 Mpa, de préférence supérieur ou égal à 300 MPa et/ou un pouvoir amortissant tg$\delta$ supérieur ou égal à 0,4, de préférence supérieur ou égal à 0,6, à une température de 30 °C et à une fréquence de 1 Hz.

**29.** Nitrocellulose selon l'une quelconque des revendications 19 à 28, apte à former un film ayant une déformation à la rupture $\varepsilon_r$ supérieure ou égale à 5 %, de préférence supérieure ou égale à 15% et/ou une énergie à rupture par unité de volume $W_r$ supérieure ou égale à 0,2 J/$cm^3$, de préférence supérieure ou égale à 1J/$cm^3$ .

**30.** Procédé de préparation d'une nitrocellulose telle que définie selon l'une quelconque des revendications 19 à 29, comprenant, lorsque la chaîne hydrocarbonée R est d'origine polymérique, une réaction de greffage consistant à faire réagir directement sur tout ou partie des fonctions hydroxyles libres d'une nitrocellulose de départ, les extrémités réactives de polymères dont les chaînes constituent la chaîne hydrocarbonée R.

Office européen des brevets

# RAPPORT DE RECHERCHE EUROPEENNE

Numéro de la demande
EP 03 10 0551

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | US 4 683 007 A (HOROWITZ ET AL.)<br>28 juillet 1987 (1987-07-28)<br>* abrégé *<br>* colonne 2, ligne 3 - ligne 10 *<br>* colonne 3, ligne 29 - ligne 38 *<br>* revendications *<br>--- | 1-10,<br>12-18,30 | C08B5/04<br>C08B13/00<br>C08B15/00<br>C08B15/06<br>A61K7/043<br>A61K7/48 |
| X | DATABASE CHEMABS 'en ligne!<br>chemical abstracts service, Columbus, Ohio, US;<br>"Aqueous enamels containing carboxyalkyl cellulose nitrates and polymethyacrylates for nails."<br>retrieved from STN<br>Database accession no. 134:120596<br>XP002225100<br>* abrégé *<br>& JP 2001 019892 A (ASAHI CHEMICAL INDUSTRY CO. LTD)<br>23 janvier 2001 (2001-01-23)<br>--- | 1,4-10,<br>16-18 | |
| | | | **DOMAINES TECHNIQUES RECHERCHES (Int.Cl.7)** |
| A | FR 1 461 812 A (CL. DUFOUR ET SEAC)<br>9 décembre 1966 (1966-12-09)<br>* page 1, colonne de gauche, ligne 12 - ligne 22 *<br>* page 5, colonne de droite, ligne 9 - ligne 36 *<br>* revendications *<br>--- | 1,4,7,19 | C08B<br>A61Q<br>A61K |
| A | M. STRAUSS ET AL.: "The Preparation and Characterization of Picryl Nitrocellulose. Formation of Anionic Sigma Complexes in a Polymeric System"<br>TETRAHEDRON LETTERS,<br>vol. 28, no. 2, 1987, pages 159-162,<br>XP001149104<br>uk<br>* page 161 *<br>---<br>-/-- | 19 | |

Le présent rapport a été établi pour toutes les revendications

| Lieu de la recherche | Date d'achèvement de la recherche | Examinateur |
|---|---|---|
| LA HAYE | 6 juin 2003 | Mazet, J-F |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

Office européen des brevets

# RAPPORT DE RECHERCHE EUROPEENNE

Numéro de la demande
EP 03 10 0551

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A | DATABASE CHEMABS 'en ligne!<br>chemical abstracts service, Columbus, Ohio, US;<br>"Acryloylated nitrocellulose"<br>retrieved from STN<br>Database accession no. 82:45464<br>XP002225101<br>* abrégé *<br>& CS 153 362 B (SIMECEK ET AL.)<br>25 février 1974 (1974-02-25)<br>--- | 19 | |
| A | NL 274 196 A (SHELL INTERNATIONALE RESEARCH MAATSCHAPPIJ NV)<br>10 septembre 1964 (1964-09-10)<br>* le document en entier *<br>--- | 19-30 | |
| A | GB 1 120 373 A (IMPERIAL CHEMICAL INDUSTRIES LIMITED)<br>17 juillet 1968 (1968-07-17)<br>* page 1, ligne 13 - page 2, ligne 42 *<br>* exemples 1,2,6,9,12 *<br>--- | 19-30 | DOMAINES TECHNIQUES RECHERCHES (Int.Cl.7) |
| A | PATENT ABSTRACTS OF JAPAN<br>vol. 016, no. 152 (C-0929),<br>15 avril 1992 (1992-04-15)<br>& JP 04 007318 A (DAICEL CHEM IND LTD),<br>10 janvier 1992 (1992-01-10)<br>* abrégé *<br>----- | 19 | |

Le présent rapport a été établi pour toutes les revendications

| Lieu de la recherche | Date d'achèvement de la recherche | Examinateur |
|---|---|---|
| LA HAYE | 6 juin 2003 | Mazet, J-F |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE RELATIF A LA DEMANDE DE BREVET EUROPEEN NO.** EP 03 10 0551

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

06-06-2003

| Document brevet cité au rapport de recherche | Date de publication | Membre(s) de la famille de brevet(s) | Date de publication |
|---|---|---|---|
| US 4683007 A | 28-07-1987 | AUCUN | |
| JP 2001019892 A | 23-01-2001 | AUCUN | |
| FR 1461812 A | 25-02-1966 | BE 690971 A | 16-05-1967 |
| | | LU 52561 A | 09-02-1967 |
| | | NL 6617389 A | 12-06-1967 |
| CS 153362 B | 25-02-1974 | CS 153362 B1 | 25-02-1974 |
| NL 274196 A | | AUCUN | |
| GB 1120373 A | 17-07-1968 | BE 699299 A | 30-11-1967 |
| | | NL 6707586 A | 01-12-1967 |
| JP 04007318 A | 10-01-1992 | AUCUN | |

EPO FORM P0460

Pour tout renseignement concernant cette annexe : voir Journal Officiel de l'Office européen des brevets, No.12/82